# EUROPEAN PATENT APPLICATION

(11) **EP 1 437 152 A1**
(43) Date of publication of application: **14.07.2004**
(21) Application number: 04468001.5
(22) Date of filing: 08.01.2004
(51) Int. Cl.: A61M 11/04

(54) **Thermal inhalator with protective lid**

(30) Priority: 10.01.2003 SI 200300004
(71) Applicant: Bremed d.o.o. Breginj, 5223 Breginj (SI)
(72) Inventor: Tonkli, Pavel, 5223 Breginj (SL)
(74) Representative: Kraljic, Janez

(57) **Abstract**

Thermal inhalator with protective lid according to this invention has such protective lid that protects the user of the thermal inhalator against burn injuries caused by hot parts of the thermal inhalator. Furthermore, the construction of the protective lid physically ensures proper distance between the thermal inhalator and the user of the thermal inhalator, and thus prevents burn injuries caused by inhaling too hot vapour.

Protective lid 2 of the thermal inhalator is placed on the heating reservoir 1 in such a way, that it covers all hot parts of the thermal inhalator, especially hot parts at the safety valve cap, nozzle with tube attachment and outlet. Protective lid also has a rigid distance holder 3, which spreads itself over the nozzle with tube attachment, and hot outlet 7, to which a flexible plastic tube 6 is attached. The length of the rigid distance holder 3 of the protective lid 2 is determined so, that the vapour temperature at the exit from the plastic tube 6 is suitable for inhalation. Therefore, the rigid distance holder 3 of the protective lid 2 with its length prevents the user of the thermal inhalator to get to close to the device, and thus also prevents the user from getting injured by inhaling too hot vapour.

## Description

Subject of this invention is a thermal inhalator with protective lid, especially with such protective lid, that protects the user of the thermal inhalator against physical burn injuries, which could be caused by getting in contact with hot parts of the thermal inhalator. The construction of the protective lid of the thermal inhalator, especially its rigid distance holder, ensures appropriate distance between the thermal inhalator and the entrance into the respiratory system of the user of the thermal inhalator. In this way burn injuries that could be caused by inhaling too hot vapour are prevented.

Technical problem, which is solved by the described invention, is such type of construction of the thermal inhalator, that ensures the safety of the user, especially safety of the user against burns, which could be caused by getting in contact with hot parts of the thermal inhalator. Furthermore, the construction of the protective lid of the thermal inhalator physically ensures proper distance between the user of the thermal inhalator and the thermal inhalator itself.

When using the thermal inhalator without protected hot parts, the user of the thermal inhalator could hurt himself; especially large is the danger of burn injuries on hot parts of the thermal inhalator. Such danger is even more pronounced, when the thermal inhalator is used by patients, children or elderly persons. For the safe use of the thermal inhalator it is also necessary to ensure, that the vapour, which is generated by the thermal inhalator, has a suitable temperature, so that it could not cause any burn injuries.

Until now most of the thermal inhalators do not have thermal protection at least at some of the parts, so the manufacturers are prescribing how to use inhalators, especially required minimum distance between the user of the thermal inhalator and the device itself. To ensure safe use, manufacturers recommend that the use of the thermal inhalator is monitored by a trained person. Such person takes care, that the thermal inhalator is used in accordance with manufacturer's instructions, and above all, that a proper distance between the user of the thermal inhalator and the device itself is ensured. This distance, e.g. 0,5 m, ensures that there will not come to any burn injuries. Professional staff in medical institutions or adult persons at home use of thermal inhalator should take care of ensuring proper safety distance.

Vapour, which comes out of the operating thermal inhalator, usually still has a high temperature at the exit from the outlet, and therefore presents a danger of causing burn injuries to the user of the thermal inhalator. To achieve a suitable vapour temperature manufacturers prescribe minimum distance between the outlet and the entrance into the respiratory system of the user of the inhalator. To ensure this distance a presence of a professionally trained person was needed again, especially when the thermal inhalator was used by patients, children or elderly persons.

Therefore, until now, the problem has been solved by prescribing how to use the device safely, and not by technical protection of the user, as it is provided with the thermal inhalator with protective lid, presented in this invention.

In accordance with this invention the problem of safe use of the thermal inhalator is solved by making of such thermal inhalator with protective lid, that has a protective lid of the thermal inhalator formed out of a thermo-insulating plastic material. The protective lid is formed in such a way, that it protects all hot parts of the thermal inhalator. Protective lid also has a rigid distance holder, which spreads itself over a nozzle with tube attachment, over an outlet and a plastic tube. Distance holder with its length also ensures proper physical distance between the user of the device and the thermal inhalator, and thus ensures a suitable temperature of the vapour, which is inhaled by the user of the thermal inhalator.

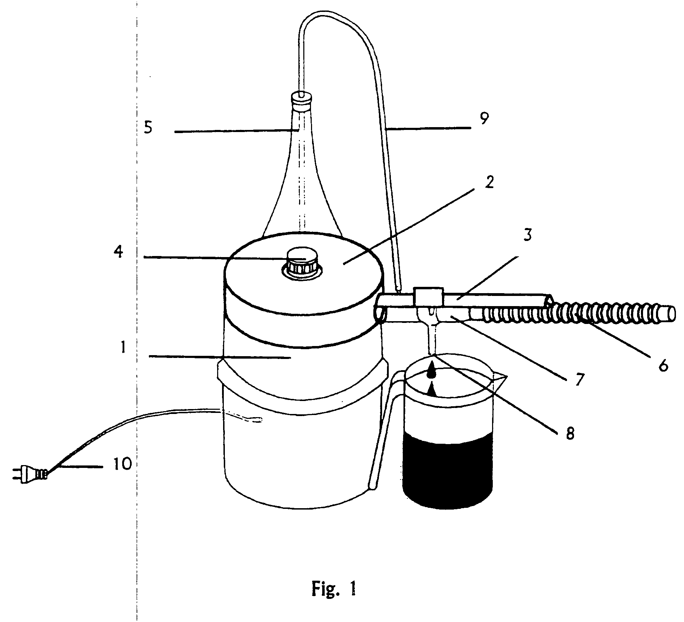

The invention is described on the example for production, and in the figures, which show
Figure 1 shows the whole thermal inhalator with protective lid.
Figure 2 shows protective lid of the inhalator.

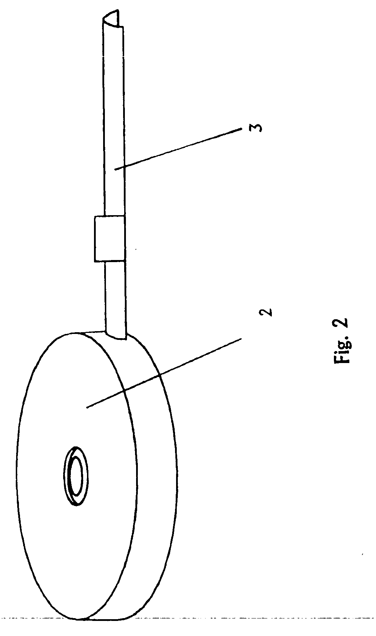

Thermal inhalator with protective lid consists of heating reservoir 1 with safety valve cap 4, protective lid 2 of the thermal inhalator with rigid distance holder 3, outlet 7 with drain opening 8, flexible plastic tube 6, bottle 5, and plastic tube 9 with plug, and power supply cable 10. Figure 2 shows preferred construction of the protective lid.

Protective lid 2 of the thermal inhalator is placed on the heating reservoir 1 in such-a way, that it covers all hot parts of the thermal inhalator, especially hot parts at the safety valve cap, nozzle with tube attachment and outlet. Protective lid also has a rigid distance holder 3, which covers nozzle with tube attachment, and hot outlet 7, to which a flexible plastic tube 6 is attached. The length of the rigid distance holder 3 of the protective lid 2 is determined so, that the vapour temperature at the exit from the plastic tube 6 is suitable for inhalation. Therefore, the rigid distance holder 3 of the protective lid 2 with its length prevents the user of the thermal inhalator to get to close to the device, and thus also prevents the user from getting injured by inhaling too hot vapour.

## Claims

1. Thermal inhalator with protective lid,
**characterized in that**
the lid (2) of the thermal inhalator is formed out of a thermo-insulating plastic material in such a way, that it protects all hot parts of the thermal inhalator, and **in that** the protective lid (2) has a rigid distance holder (3), which spreads itself over a nozzle with tube attachment, and over an outlet (7), and a plastic tube (6), where the rigid distance holder with its length ensures proper distance between the user of the device and the thermal inhalator, and thus ensures a suitable temperature of the vapour, which is inhaled by the user of the thermal inhalator.
